# EUROPEAN PATENT APPLICATION

(11) **EP 0 976 404 A2**
(43) Date of publication of application: **02.02.2000**
(21) Application number: 99305938.5
(22) Date of filing: 26.07.1999
(51) Int. Cl.: A61K 45/06, A61K 31/135, A61K 31/445

(54) **A pharmaceutical composition for the prevention and treatment of diseases of cognitive dysfunction in a mammal**

(30) Priority: 30.07.1998 US 94653 P
(71) Applicant: Pfizer Products Inc., Groton, Connecticut 06340 (US)
(72) Inventor: Dasilva-Jardine, Paul A., Groton, Connecticut 06340 (US)
(74) Representative: Ruddock, Keith Stephen

(57) **Abstract**

Pharmaceutical compositions for the treatment of diseases involving cognitive dysfunction in a mammal comprising an estrogen agonist or antagonist or a pharmaceutically acceptable salt thereof; an acetyl cholinesterase inhibitor or a pharmaceutically acceptable salt thereof; and a pharmaceutically acceptable carrier. The estrogen agonists or antagonists and acetylcholinesterase inhibitors are present in amounts that render the composition effective in the treatment of diseases of cognitive dysfunction including Alzheimer's Disease and Dementia. The method of using these composition is also disclosed. The compositions may help memory inhancement.

## Description

### Background of the Invention

The present invention relates to a pharmaceutical compositions for the prevention and treatment of diseases of cognitive dysfunction in a mammal comprising estrogen agonists or antagonists, acetylcholine esterase inhibitors and a pharmaceutically acceptable carrier. The pharmaceutical compositions are useful in enhancing memory in patients suffering from Dementia and Alzheimer's Disease.

Alzheimer's Disease (AD) is a degenerative brain disorder characterized clinically by progressive loss of memory, cognition, reasoning, judgment and emotional stability that gradually leads to profound mental deterioration and ultimately death. AD is a common cause of progressive mental failure (dementia) in aged humans and is believed to represent the fourth most common medical cause of death in the United States. AD has been observed in varied races and ethnic groups worldwide and presents a major present and future public health problem. The disease is currently estimated to affect about two to three million individuals in the United States alone. To date, AD has proven to be incurable and will increase worldwide as the human lifespan increases.

Alzheimer's Disease is associated with degeneration of cholinergic neurons in the basal forebrain that play a fundamental role in cognitive functions, including memory. Becker et al., Drug Development Research, 12, 163-195 (1988). As a result of such degeneration, patients suffering from the disease exhibit a marked reduction in acetylcholinesterase activity and choline uptake.

It is know that acetylcholinesterase inhibitors are effective in enhancing cholinergic activity and useful in improving the memory of Alzheimer's patients. By inhibiting the acetylcholinesterase enzyme, these compounds increase the level of the neurotransmitter acetylcholine, in the brain and thus enhance memory. Becker et al., supra, report that behavioral changes following cholinesterase inhibition appear to coincide with predicted peak levels of acetylcholine in the brain. They also discuss the efficacy of the three known acetylcholinesterase inhibitors pysostigmine, metrifonate, and tetrahydroaminoacridine.

U.S. Patent Application Ser. No. 07/639,614 filed Jan 10, 1991, U.S. Patent Application Ser. No. 07/676,918 filed March 28, 1991, U.S. Patent No. 5,750,542 and U.S. Patent No. 5,574,046 all of which are assigned in common with the present application, also refer to heteroaryl amine acetylcholinesterase inhibitors and are incorporated by reference.

Another worldwide disease of aging is osteoporosis, a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,00 spine, 250,000 hip and 240,000 wrist fractures annually. These cost the nation over $10 billion. Hip fractures are the most serious, with 55-20% of patients dying within one year, and over 50% of survivors being incapacitated.

The elderly are at greatest risk of osteoporosis, and the problem is therefore predicted to increase significantly with the aging of the population. Worldwide fracture incidence is forecast to increase three-fold over the next 60 years, and one study estimates that there will be 4.5 million hip fractures worldwide by 2050.

Women are at greater risk of osteoporosis than men. Women experience a sharp acceleration of bone loss during the five years following menopause. Other factors that increase the risk include smoking, alcohol abuse, a sedentary lifestyle and low calcium intake.

Estrogen is the agent of choice in preventing osteoporosis or post menopause bone loss in women; it is the only treatment which unequivocally reduces fractures. Several studies in humans also indicate that estrogen usage protects against and reverses a decline in cognitive function. Estrogen also appears to improve cognitive function in patients with clinical senile dementias such as Alzheimer's Disease. This was reviewed recently by K. Yaffe, G. Sawaya, I. Lieberburg, and D. Grady in the Journal of the American Medical Association, Vol. 279 No. 9 p-688-695 (1998). Part of estrogens beneficial effects on cognition and dementia is through its effects on the cholinergic system which plays an important role in the etiology of cognition and dementia. Acetylcholine esterase inhibitors appears to effect cognition and dementia by exerting its beneficial effects on the same cholinergic systems. Thus a combination of estrogen with acetylcholine esterase inhibitors may have significantly greater beneficial effects. However usage of estrogen is also associated with several untoward side effects including an increased risk of breast and uterine cancer. Estrogen agonists or antagonists, like those included in this application, may maintain many of estrogens neuroprotective effects but be devoid of its adverse effects on other tissues like the breast and uterine.

Estrogen agonists or antagonists are herein defined as chemical compounds capable of binding to the estrogen receptor site in mammalian tissue and blocking the actions of estrogen in one or more tissues. Estrogen agonists, such as droloxifene, mimic estrogen's beneficial effects without some of its adverse effects. The combination of an estrogen agonist or antagonist and a acetylcholinesterase inhibitor will have benefits for the treatment of cognitive disorders, memory enhancement and treatment of Alzheimer's Disease or Dementia.

### Summary of the Invention

This invention provides a pharmaceutical composition for the treatment of diseases of cognitive dysfunction in a mammal, comprising an estrogen agonist or antagonist or a pharmaceutically acceptable salt thereof, an acetylcholinesterase inhibitor or a pharmaceutically acceptable salt thereof, and a pharmaceutically acceptable carrier, wherein the estrogen agonist or antagonist and the acetylcholinesterase inhibitor are present in amounts that render the composition effective in the treatment of diseases of cognitive dysfunction.

This invention also relates to the use of such compositions for the treatment of conditions which present with loss of cognitive function including Alzheimer's Disease or Dementia in mammals (e.g., humans).

A preferred estrogen agonist or antagonist is droloxifene: (phenol, 3-(1-(4-(2-dimethylamino)ethoxy)phenyl)-2-phenyl-1-butenyl)-, (E)-) and related compounds which are disclosed in U.S. patent 5,047,431, the disclosure of which is incorporated herein by reference.

Another related preferred compound is 3-(4-(1,2-diphenyl-butyl-1-enyl)-phenyl)acrylic acid, which is disclosed in Willson et al., Endocrinology, 1997, 138, 3901-3911, the disclosure of which incorporated herein by reference.

Another related preferred compound is tamoxifen: (ethanamine,2-(-4(1,2-diphenyl-1-butenyl)phenoxy)-N,N-dimethyl, (Z)-2-, 2-hydroxy-1 ,2,3-propanetricarboxylate(1:1)) and related compounds which are disclosed in U.S. patent 4,536,516, the disclosure of which is incorporated herein by reference.

Another related preferred compound is 4-hydroxy tamoxifen which is disclosed in U.S. patent 4,623,660, the disclosure of which is incorporated herein by reference.

Another preferred estrogen agonist or antagonist is raloxifene: (methanone, (6-hydroxy2-(4-hydroxyphenyl)benzo[b]thien-3-yl)(4-(2-(I-piperidinyl)ethoxy)phenyl)hydrochloride) which is disclosed in U.S. patent 4,418,068, the disclosure of which is incorporated herein by reference.

Another preferred estrogen agonist or antagonist is toremifene: (ethanamine, 2-(4(4-chloro-1,2-diphenyl-1-butenyl)phenoxy)-N,N-dimethyl-, (Z)-, 2-hydroxy-1,2,3-propanetricarboxylate (1:1) which is disclosed in U.S. patent 4,996,225, the disclosure of which is incorporated herein by reference.

Another preferred estrogen agonist or antagonist is centchroman: 1-(2-((4(-methoxy-2,2, dimethyl-3-phenyl-chroman-4-yl)-phenoxy)-ethyl)-pyrrolidine, which is disclosed in U.S. patent 3,822,287, the disclosure of which is incorporated herein by reference. Also preferred is levormeloxifene: 1-{2-[4-(7-Methoxy-2,2-dimethyl-3-phenyl-chroman-4-yl)-phenoxy]-ethyl}pyrrolidine(-)isomer levorotatory.

Another preferred estrogen agonist or antagonist is idoxifene: (E)-1-(2-(4-(I-(4-iodo-phenyl)-2-phenyl-but-l-enyl)-phenoxy)-ethyl)-pyrrolidinone, which is disclosed in U.S. patent 4,839,155, the disclosure of which is incorporated herein by reference.

Another preferred estrogen agonist or antagonist is 2-(4-methoxy-phenyl)-3-[4-(2-piperidin-1-yl-ethoxy)-phenoxy]- benzo[b]thiophen-6-ol which is disclosed in U.S. Patent No. 5,488,058, the disclosure of which is incorporated herein by reference.

Another preferred estrogen agonist or antagonist is 6-(4-hydroxy-phenyl)-5-(4-(2-piperidin-1-yl-ethoxy)-benzyl)-naphthalen-2-ol which is disclosed in U.S. patent 5,484,795, the disclosure of which is incorporated herein by reference.

Another preferred estrogen agonist or antagonist is (4-(2-(2-aza-bicyclo[2.2. 1]hept-2-yl)-ethoxy)-phenyl)-(6-hydroxy-2-(4-hydroxy-phenyl)-benzo[bjthiophen-3-yl)methanone which is disclosed, along with methods of preparation, in PCT publication no. WO 95/10513 assigned to Pfizer Inc.

Other preferred estrogen agonist or antagonists include compounds as described in commonly assigned U.S. patent 5,552,412, the disclosure of which is incorporated herein by reference. Especially preferred compounds described therein are:
cis-6-(4-fluoro-phenyl)-5-(4-(2-piperidin-1-yl-ethoxy)-phenyl)-5,6,7,8-tetrahydronaphthalene-2-ol;
(-)-cis-6-phenyl-5-(4-(2-pyrrolidin-1-yl-ethoxy)-phenyl)-5,6,7,8-tetrahydronaphthalene-2-ol;
-cis-6-phenyl-5-(4-(2-pyrrolidin-1-yl-ethoxy)-phenyl)-5,6,7,8-tetrahydronaphthalene-2-ol;
cis-1-(6'-pyrrolodinoethoxy-3'-pyridyl)-2-phenyl-6-hydroxy-1,2,3,4tetrahydronaphthalene;
1-(4'-pyrrolidinoethoxyphenyl-2-(4"-fluorophenyl)-6-hydroxy-1,2,3,4-tetrahydroisoquinoline;
cis-6-(4-hydroxyphenyl)-5-(4-(2-pipedin-1-yl-ethoxy)-phenyl)-5,6,7,8-tetrahydronaphthalene-2-ol; and
1 -(4'-pyrrolidinolethoxyphenyl)-2-phenyl-6-hydroxy-1,2,3,4-tetrahydroisoquinoline.

Other estrogen agonists or antagonists are described in U.S. patent 4,133,814 (the disclosure of which is incorporated herein by reference). U.S. patent 4,133,814 discloses derivatives of 2-phenyl-3-aroyl-benzothiophene and 2-phenyl-3-aroylbenzothiophene-1-oxide.

Preferred acetylcholine esterase inhibitors include Donepezil which is 2-(1-Benzylpiperidin-4-ylmethyl)-5,6-dimethoxy-indan-1-one; 3-(1-Benzyl-piperidin-4-yl)-1-(2methylbenzothiazol-6-yl)-propane-1-one and 3-[2-(1-Benzyl-piperidin 4-yl, ethyl]-5,7-dihydro-1-oxa-2,7 diaza-s-indacene-6-one

Another aspect of this invention is a method for treating mammals which present with loss of cognitive function, comprising administering to a mammal an estrogen agonist or antagonist or a pharmaceutically acceptable salt thereof; and an acetylcholinesterase inhibitor or a pharmaceutically acceptable salt thereof; wherein the estrogen agonist or antagonist and the acetyl chlolinesterase inhibitor are present in amounts that render the composition effective in the treatment of diseases of cognitive dysfunction.

Preferred estrogen agonist or antagonists in this method include droloxifene, raloxifene, tamoxifen, 4-hydroxy-tamoxifen, idoxifene, levomerloxifene, toremifene, centchroman,
(-)-*Cis*-6-phenyl-5-[4-(2-pyrrolidin-1-yl-ethoxy)-phenyl]-5,6,7,8-tetrahydronaphthalene-2-ol;
*Cis*-6-(4-fluoro-phenyl)-5-[4-(2-piperidin-1-yl-ethoxy)-phenyl]-5,6,7,8-tetrahydronaphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-[6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-[4-(2-piperidin-1-yl-ethoxy)-phenyl]-5,6,7,8-tetrahydronaphthalene-2-ol; and
1 -(4'-Pyrrolidinolethoxyphenyl)-2-phenyl-6-hydroxy-1,2,3,4-tetrahydroisoquinoline.

Other estrogen agonists or antagonists are described in U.S. patent 4,133,814 (the disclosure of which is incorporated herein by reference). U.S. patent 4,133,814 discloses derivatives of 2-phenyl-3-aroyl-benzothiophene and 2-phenyl-3-aroylbenzothiophene-l-oxide.

Preferred acetylcholine esterase inhibitors include Donepezil which is 2-(1-Benzyl-piperidin-4-ylmethyl)-5,6-dimethoxy-indan-1-one; 3-(1-Benzyl-piperidin-4-yl)-1-(2-methylbenzothiazol-6-yl)-propan-1-one; and 3-[2-(1-Benzyl-piperidin-4-yl)-ethyl]-5,7-dihydro-1-oxa-2,7-diaza-s-indacene-6-one

A preferred aspect of this method is wherein the estrogen agonist or antagonist is droloxifene: (-)-*Cis*-6-phenyl-5-[4-(2-pyrrolidin-1-yl-ethoxy)-phenyl]-5,6,7,8-tetrahydronaphthalene-2-ol.

Another preferred aspect of this method is wherein the condition which presents with low cognition is Alzheimer's Disease.

Another preferred aspect of this method is wherein estrogen agonist or antagonist and acetyl cholinesterase inhibitor are administered substantially simultaneously.

Those skilled in the art will recognize that the term cognitive dysfunction which is sometimes (although not strictly correctly) referred to as Alzheimer's disease or Dementia.

The term treating", "treat" or "treatment" as used herein includes preventive (e.g., prophylactic) and palliative treatment.

The chemist of ordinary skill will recognize that certain compounds of this invention will contain one or more atoms which may be in a particular stereochemical or geometric configuration, giving rise to stereoisomers and configurational isomers. All such isomers and mixtures thereof are included in this invention. Hydrates of the compounds of this invention are also included.

The chemist of ordinary skill will recognize that certain combinations of heteroatom-containing substituents listed in this invention define compounds which will be less stable under physiological conditions (e.g. those containing acetal or animal linkages). Accordingly, such compounds are less preferred.

### DETAILED DESCRIPTION OF THE INVENTION

A mammalian estrogen agonist or antagonist or a pharmaceutically acceptable salt of the forgoing compounds may be used in this invention. The term estrogen agonist or antagonist refers to chemical compounds which bind with the estrogen receptor sites in mammalian tissue and block the actions of estrogen in one or more tissues.

An acetyl cholinesterase inhibitor or a pharmaceutically acceptable salt of the foregoing compounds such as Donepezil which is 2-(1-Benzyl-piperidin-4-ylmethyl)-5,6-dimethoxy-indan-1-one; 3-(1-Benzyl-piperidin-4-yl)-1-(2-methyl-benzothiazol-6-yl)-propan-1-one and 3-[2-(1-Benzyl-piperidin-4-yl)-ethyl]-5,7-dihydro-1-oxa-2,7-diaza-s-indacene-2-one and compounds in U.S. Patent Nos. 5,7,50,542 and 5,574,046 may be used in this invention.

In general, the compounds of this invention can be made by processes which include processes known in the chemical arts, particularly in light of the description contained herein.

Some of the preparation methods useful for making the compounds of this invention may require protection of remote functionality (i.e., primary amine, secondary amine, carboxyl). The need for such protection will vary depending on the nature of the remote functionality and the conditions of the preparation methods. The need for such protection is readily determined by one skilled in the art. The use of such protection/deprotection methods is also within the skill in the art. For a general description of protecting groups and their use, see T.W. Greene, Protective Groups in Organic Synthesis, John Wiley & Sons, New York, 1991. The starting materials and reagents for the compounds of this invention are also readily available or can be easily synthesized by those skilled in the art using conventional methods of organic synthesis. For example, many of the compounds used herein are related to, or are derived from compounds found in nature, in which there is a large scientific interest and commercial need, and accordingly many such compounds are commercially available or are reported in the literature or are easily prepared from other commonly available substances by methods which are reported in the literature. Such compounds include, for example, prostaglandins.

Some of the compounds of this invention are ionizable at physiological conditions. Thus, for example some of the compounds of this invention are acidic and they form a salt with a pharmaceutically acceptable cation. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the compounds of this invention are basic, and they form a salt with a pharmaceutically acceptable anion. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, when the compounds of this invention form hydrates or solvates they are also within the scope of the invention.

The utility of the compounds of the present invention as medical agents in the treatment of conditions which present with low cognitive function (e.g., Alzheimer's, Dementia) in mammals (e.g. humans) is demonstrated by the activity of the compounds of this invention in conventional assays and the in vitro assays described below in vitro estrogen receptor binding assay; and acetylcholinesterase inhibitor protocol. Such assays also provide a means whereby the activities of the compounds of this invention can be compared between themselves and with the activities of other known compounds. The results of these comparisons are useful for determining dosage levels in mammals, including humans, for the treatment of such diseases.

### Acetyl Cholinesterase inhibitor Protocol

Inhibition of Acetylcholinesterase (AChE) and Butyrylcholinesterase (BuChE). The method of Ellman, GL.; Courtney, K.D.; Andres, V., Jr.; Featherstone, R.M. A New and Rapid Colorimetric Determination of Acetylcholinesterase Activity. *Biochem. Pharmacol.* **1961**, 7, 88-95 was followed. The assay solution consists of a 0.1 M sodium phosphate buffer, pH 8.0, with the addition of 100 µM tetraisopropypyrophosphoramide (iso-OMPA), 100 µM 5.5'-dithiobis(2-nitrobenzoic acid) (DTNB), 0.02 units/mL AChE (Sigma Chemical Col, from human erythrocytes) and 200 µM acetylthiocholine iodide. The final assay volume was 0.25 mL. Test compounds were added to the assay solution prior to enzyme addition, whereupon a 20-min preincubation period with enzyme was followed by addition of substrate. Changes in absorbance at 412 nM were recorded for 5 min. The reaction rates were compared, and the percent inhibition due to the presence of test compounds was calculated.

Inhibition of butyrylcholinesterase was measured as described above for AChE by omitting addition of *iso*-OMPA and substitution 0.02 units/mL of BuChE (Sigma Chemical Co., from horse serum) and 200 µM butyrylthiocholine for enzyme and substrate, respectively.

*In vivo* Microdialysis. Male Sprague-Dawley rats were implanted in the corpus striatum with guide cannulae and dialysis probes (Bioanalytical Systems, West Lafayette, IN) and superfused at a rate 3 mL/minute. The dialysis fluid was a Ringer's buffer (pH 7.2) containing 500 nM physostigmine to reduce degradation of Ach by AChE. Fractions (60 µl) were collected every 20 minutes for 2 hours before drug administration and for 3 hours following oral administration of drug. Samples (50 µl) were used directly for HPLC analysis of Ach content as described above. Basal Ach release was defined as the average Ach content in the three fractions just prior to drug administration. Ach content in all fractions was converted to a percentage of these basal control values.

### In Vitro Estrogen Reeptor Binding Assay

An *in vitro* estrogen receptor binding assay, which measures the ability of the estrogen agonist or antagonist compounds of the present invention to displace [3H]-estradiol from human estrogen receptor obtained by recombinant methods in yeast, is used to determine the estrogen receptor binding affinity of the compounds of this invention. The materials used in this assay are: (1) Assay buffer, TD-0.3 (containing 10 Nm Tris, pH 7.6, 0.3 M potassium chloride and 5 mM Dithiothreitol (DTT) (Sigma Co.), pH 7.6); (2) The radioligand used is [3H]-estradiol obtained from New England Nuclear; (3) the cold ligand used is estradiol obtained from Sigma (4) recombinant human estrogen receptor, hER.

A solution of the compound being tested is prepared in TD-0.3 with 4% DMSO and 16% ethanol. The tritiated estradiol is dissolved in TD-0.3 such that the final concentration in the assay is 5nM. The hER is also diluted with TD-0.3 such that 4-10 pg of total protein is in each assay well. Using microtitre plates, each incubate receives 50 ul of cold estradiol (nonspecific binding) or the compound solution, 20 uL of the tritiated estradiol and 30 ul of hER solutions. Each plate contains in triplicate total binding and varying concentrations of the compound. The plates are incubated overnight at 4°C. The binding reaction is then terminated by the addition and mixing of 100 mL of 3% hydroxylapatite in 10 mM tris, pH 7.6 and incubation for 15 minutes at 4°C. The mixtures are centrifuged and the pellet washed four times with 1% Triton-X100 in 10 mM Tris, pH 7.6. The hydroxylapatite pellets are suspended in Ecoscint A and radioactivity is assessed using beta scintigraphy. The mean of all triplicate data points (counts per minute, cpm's) is determined. Specific binding is calculated by subtracting nonspecific cpm's (defined as counts that remain following separation of reaction mixture containing recombinant receptor, radioligand, and excess unlabeled ligand) from total bound cpm's (defined as counts that remain following the separation of reaction mixture containing only recombinant receptor, radioligand). Compound potency is determined by means of IC50 determinations (the concentration of a compound needed to inhibition 50% of the of the total specific tritiated estradiol bound). Specific binding in the presence of varying concentrations of compound is determined and calculated as percent specific binding of total specific radioligand bound. Data are plotted as percent inhibition by compound (linear scale) versus compound concentration (log scale).

Administration of the compositions of this invention can be via any method which delivers a compound of this invention systemically and/or locally. These methods include oral routes, parenteral, intraduodenal routes, etc. Generally, the compounds of this invention are administered orally, but parenteral administration (e.g., intravenous, intramuscular, subcutaneous or intramedullary) may be utilized, for example, where oral administration is inappropriate for the instant target or where the patient is unable to ingest the drug. The two different compounds of this invention can be co-administered simultaneously or sequentially in any order, or a single pharmaceutical composition comprising an estrogen agonist or an antagonist as described above and an acetylcholinesterase inhibitor as described above in a pharmaceutically acceptable carrier can be administered.

The amount and timing of compounds administered will, of course, be dependent on the subject being treated, on the severity of the affliction, on the manner of administration and on the judgement of the prescribing physician. Thus, because of patient to patient variability, the dosages given below are a guideline and the physician may titrate doses of the drug to achieve the activity (e.g., increasing cognitive process) that the physician considers appropriate for the individual patient. In considering the degree of activity desired, the physician must balance a variety of factors such as cognitive function, age of the patient, presence of preexisting disease, as well as presence of other diseases (e.g., cardiovascular). For example, the administration of an estrogen agonist or antagonist can provide cardiovascular benefits particularly, for post-menopausal women. The following paragraphs provide preferred dosage ranges for the various components of this invention.

In general an effective dosage for the estrogen agonists or antagonists are in the range of 0.01 to 200 mg/kg/day, preferably 0.05 to 100 mg/kg/day.

In particular, an effective dosage for idoxifene is in the range of 0.02 to 2 mg/kg/day

In particular, an effective dosage for levomerloxifene is in the range of 0.05 to 10 mg/kg/day.

In particular, an effective dosage for droloxifene is in the range of 0.1 to 40 mg/kg/day, preferably 0.1 to 5 mg/kg/day.

In particular, an effective dosage for raloxifene is in the range of 0.1 to 100 mg/kg/day, preferably 0.1 to 10 mg/kg/day.

In particular, an effective dosage for tamoxifen is in the range of 0.1 to 100 mg/kg/day, preferably 0.1 to 5 mg/kg/day.

In particular, an effective dosage for
*Cis*-6-(4-fluoro-phenyl)-5-[4-(2-piperidin-1-yl-ethoxy)-phenyl]-5,6,7,8-tetrahydro-naphthalene-2-ol;
(-)-*Cis*-6-phenyl-5-[4-(2-pyrrolidin-1-yl-ethoxy)-phenyl]-5,6,7,8-tetrahydro-naphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-(6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-[4-(2-piperidin-1-yl-ethoxy)-phenyl]-5,6,7,8-tetrahydro-naphthalene-2-ol; or
1-(4'-Pyrrolidinolethoxyphenyl)-2-phenyl-6-hydroxy-1,2,3,4-tetrahydroisoquinoline is in the range of 0.0001 to 100 mg/kg/day, preferably 0.001 to 10 mg/kg/day.

In particular, an effective dosage for 4-hydroxy tamoxifen is in the range of 0.0001 to 100 mg/kg/day, preferably 0.001 to 10 mg/kg/day.

In general an effective dosage for the acetyl cholinesterase inhibition is 0.01 to 200mg/kg/day. More spectic dosages are as follows:
(1) For Donepezil the range is 0.1 to 20 mg/kg/day
(2) For 3(1-benzyl-piperidin-4yl)-1-(2methyl-benxothiazol-6-yl)propene-1-one and 3-[2-(1-Benzyl-piperidin-4-yl-ethyl]-5,7-dihydro-1-oxo-2,7-diaza-5-indacene-6-one the range is 0.01 to 5mg/kg/day

The compositions of the present invention are generally administered in the form of a pharmaceutical composition comprising at least one of the compounds of this invention together with a pharmaceutically acceptable vehicle or diluent. Thus, the compounds of this invention can be administered individually or together in any conventional oral, parenteral or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the disease/condition of the subject being treated.

## Claims

1. A pharmaceutical composition for the treatment of diseases involving cognitive dysfunction in a mammal comprising:
a an estrogen agonist or antagonist or a pharmaceutically acceptable salt thereof;
b an acetyl cholinesterase inhibitor or a pharmaceutically acceptable salt thereof;
wherein the estrogen agonist or antagonist and the acetyl cholinesterase inhibitor are present in amounts that render the composition effective in the treatment of diseases of cognitive dysfunction.

2. A pharmaceutical composition as recited in claim 1 wherein the estrogen agonist or antagonist is droloxifene, raloxifene, tamoxifen, 4-hydroxy-tamoxifen, idoxifene, levomerloxifene, toremifene, centchroman,
*Cis*-6-(4-fluoro-phenyl)-5-[4-(2-piperidin-1-yl-ethoxy)-phenyl]-5,6,7,8-tetrahydro-naphthalene-2-ol;
(-)-*Cis*-6-phenyl-5-[4-(2-pyrrolidin-1-yl-ethoxy)-phenyl]-5,6,7,8-tetrahydro-naphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-[6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-[4-(2-piperidin-1-yl-ethoxy)-phenyl]-5,6,7,8-tetrahydro-naphthalene-2-ol;
1 -(4'-Pyrrolidinolethoxyphenyl)-2-phenyl-6-hydroxy-1,2,3,4-tetrahydroisoquinoline,
6-(4-hydroxy-phenyl-5-(4-(2-piperidin-1-yl-ethoxy)-benzyl-naphthal-2-ol or (4-(2-(2-azabicydo[2.2.1.] hept-2-yl)-ethoxy)-phenyl-(6-hydroxy-2-(4-hydroxy-phenyl-benzo)-6-thio; or a pharmaceutically acceptable salt of one of the foregoing compounds.

3. A pharmaceutical composition according to claim 1 wherein the acetyl cholinesterase inhibitor is selected from the group consisting of Donepezil, 3-(1-Benzyl-piperidin-4-yl)-1-(2-methyl-benzothiazol-6-yl)-propan-1-one, or 3-[2-(1-Benzyl-piperidin-4-yl)ethyl]-5,7-dihydro-1-oxa-2,7-diaza-s-indacene-6-one; or a pharmaceutically acceptable salt of one of the foregoing compounds

4. A pharmaceutical composition as recited in claim 2 wherein the estrogen agonist or antagonist is droloxifene.

5. A pharmaceutical composition according to claim 3 wherein the acetyl cholinesterase inhibitor is 3-(1-Benzyl-piperidin-4-yl)-1-(2-methyl-benzothiazol-6-yl)-propan-1-one;

6. A pharmaceutical composition according to claim 3 wherein the acetyl cholinesterase inhibitor is 3-[2-(1-Benzyl-piperidin-4-yl)-ethyl]-5,7-dihydro-1-oxa-2,7-diaza-s-indacene-6-one;

7. A pharmaceutical composition according to claim 1 wherein the estrogen agonist or antagonist is
*Cis*-6-(4-fluoro-phenyl)-5-[4-(2-piperidin-1-yl-ethoxy)-phenyl]-5,6,7,8-tetrahydro-naphthalene-2-ol;
(-)-*Cis*-6-phenyl-5-[4-(2-pyrrolidin-1-yl-ethoxy)-phenyl]-5,6,7,8-tetrahydro-naphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-[6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-[4-(2-piperidin-1-yl-ethoxy)-phenyl]-5,6,7,8-tetrahydro-naphthalene-2-ol; or
1-(4'-Pyrrolidinolethoxyphenyl)-2-phenyl-6-hydroxy-1,2,3,4-tetrahydroisoquinoline; or a pharmaceutically acceptable salt of one of the foregoing compounds thereof;

8. A pharmaceutical composition according to claim 7 wherein the acetylcholinesterase inhibitor is 3-(1-Benzyl-piperidin-4-yl)-1-(2-methyl-benzothiazol-6-yl)propan-1-one or 3-[2-(1-Benzyl-piperidin-4-yl)-ethyl]-5,7-dihydro-1-oxa-2,7-diaza-s-indacene-6-one; or a pharmaceutically acceptable salt of one of the foregoing compounds thereof.

9. A pharmaceutical composition according to claim 7 wherein the acetyl cholinesterase inhibitor is Donepezil.

10. A method for treating a mammal having a condition which presents with loss of cognitive function, comprising administering to said mammal
a. an estrogen agonist or antagonist; or a pharmaceutically acceptable salt thereof;
b. an acetyl cholinesterase inhibitor; or a pharmaceutically acceptable salt thereof;
wherein the estrogen agonist or antagonist and the acetyl cholinesterase inhibitor are present in amounts that render the composition effective in the treatment of diseases of cognitive dysfunction.

11. A method as recited in claim 10 wherein the estrogen agonist or antagonist is droloxifene, raloxifene, tamoxifen, 4-hydroxy-tamoxifen, idoxifene, centrochroman, levomerloxifene
*Cis*-6-(4-fluoro-phenyl)-5-[4-(2-piperidin-1-yl-ethoxy)-phenyl]-5,6,7,8-tetrahydro-naphthalene-2-ol;
(-)-*Cis*-6-phenyl-5-[4-(2-pyrrolidin-1-yl-ethoxy)-phenyl]-5,6,7,8-tetrahydro-naphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-[6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-(4-(2-piperidin-1-yl-ethoxy)-phenyl]-5,6,7,8-tetrahydro-naphthalene-2-ol; or
1-(4'-Pyrrolidinolethoxyphenyl)-2-phenyl-6-hydroxy-1,2,3,4-tetrahydroisoquinoline; or a pharmaceutically acceptable salt of one of the foregoing compounds thereof.

12. A method as recited in claim 11 wherein the acetyl cholinesterase inhibitor is Donepezil.

13. A method as recited in claim 12 wherein the estrogen agonist or antagonist is droloxifene.

14. A method as recited in claim 11 wherein the acetyl chlorinesterase inhibitor is 3-(1-Benzyl-piperidin-4-yl)-1-(2-methyl-benzothiazol-6-yl)-propan-1-one.

15. A method as recited in claim 11 wherein the acetyl cholinesterase inhibitor is 3-[2-(1-Benzyl-piperidin-4-yl)-ethyl]-5,7-dihydro-1-oxa-2,7-diaza-s-indacene-6-one

16. A method as recited in claim 13 wherein the condition which presents with loss of cognitive process is Alzheimer's Disease.

17. A method as recited in claim 12 wherein the estrogen agonist or antagonist is
*Cis*-6-(4-fluoro-phenyl)-5-[4-(2-piperidin-1-yl-ethoxy)-phenyl]-5,6,7,8-tetrahydro-naphthalene-2-ol;
(-)-*Cis*-6-phenyl-5-[4-(2-pyrrolidin-1-yl-ethoxy)-phenyl]-5,6,7,8-tetrahydro-naphthalene-2-ol;
*Cis*-6-phenyl-5-[4-(2-pyrrolidin-1-yl-ethoxy)-phenyl]-5,6,7,8-tetrahydro-naphthalene-2-ol;
*Cis*-1-[6'-pyrrolodinoethoxy-3'-pyridyl]-2-phenyl-6-hydroxy-1,2,3,4-tetrahydrohaphthalene;
1 -(4'-Pyrrolidinoethoxyphenyl)-2-(4"-fluorophenyl)-6-hydroxy-1,2,3,4-tetrahydroisoquinoline;
*Cis*-6-(4-hydroxyphenyl)-5-[4-(2-piperidin-1-yl-ethoxy)-phenyl]-5,6,7,8-tetrahydro-naphthalene-2-ol; or
1-(4'-Pyrrolidinolethoxyphenyl)-2-phenyl-6-hydroxy-1,2,3,4-tetrahydroisoquinoline; or a pharmaceutically acceptable salt of one of the foregoing compounds thereof

18. A method as recited in claim 14 wherein the condition which presents with the loss of cognitive process is Dementia.

19. A method as recited in claim 14 wherein the estrogen agonist or antagonist and the acetyl cholinesterase inhibitors are administered substantially simultaneously.
